# EUROPEAN PATENT APPLICATION

(11) **EP 4 693 332 A1**
(43) Date of publication of application: **11.02.2026**
(21) Application number: 24784677.7
(22) Date of filing: 11.03.2024
(51) Int. Cl.: G16H 50/00

(54) **DISEASE PREDICTION DEVICE, DISEASE PREDICTION METHOD, PROGRAM, AND RECORDING MEDIUM**

(30) Priority: 04.04.2023 JP 2023060749
(71) Applicant: NEC Solution Innovators, Ltd., Koto-ku, Tokyo 136-8627 (JP)
(72) Inventor: KATO, Shintaro, Tokyo 1368627 (JP); YASUDA, Kosuke, Tokyo 1368627 (JP)
(74) Representative: Betten & Resch
(86) International application number: PCT/JP2024/009241
(87) International publication number: WO 2024/209882

(57) **Abstract**

Provided is a disease prediction apparatus capable of finding a risk of disease onset even in daily life. A disease prediction apparatus (10) of the present disclosure includes an information acquisition part (11), a disease prediction part (12), and an information output part (13). The information acquisition part (11) acquires past non self-examination value information and current self-examination value information of a subject of disease prediction. The disease prediction part (12) predicts disease that may occur in the future in the subject of disease prediction, based on the non self-examination value information and the self-examination value information, to generate disease prediction information. The information output part (13) outputs the disease prediction information.

## Description

### INCORPORATION BY REFERENCE

This application is based upon and claims the benefit of priority from Japanese patent application No. 2023-060749, filed on April 4, 2023, the disclosure of which is incorporated herein in its entirety by reference.

### TECHNICAL FIELD

The present invention relates to a disease prediction apparatus, a disease prediction method, a program, and a recording medium.

### BACKGROUND ART

Health checkups are conducted to grasp individual health conditions, such as a risk of disease onset. Results of those checkups are utilized for health management, such as disease prevention. A technology for predicting disease based on information on health checkups is also disclosed (Patent Literature 1).

### Citation List

### Patent Literature

Patent literature 1: JP 2022-551005 A

### SUMMARY

### Technical Problem

However, examinations conducted in health checkups, complete medical checkups, and the like include not only items that can be measured on their own, but also those that cannot. In addition, it is usual to take health checkups and the like only once or twice a year, making it difficult to check one's own health condition on a daily basis.

An example object of the invention is to provide a disease prediction apparatus capable of finding a risk of disease onset even on a daily basis.

### Solution to Problem

A disease prediction apparatus according to an example aspect of the invention includes an information acquisition part that acquires past non self-examination value information and current self-examination value information of a subject of disease prediction, a disease prediction part that predicts disease that may occur in the future in the subject of disease prediction, based on the non self-examination value information and the self-examination value information, to generate disease prediction information, and an information output part that outputs the disease prediction information.

A disease prediction method according to an example aspect of the invention includes acquiring information that acquires past non self-examination value information and current self-examination value information of a subject of disease prediction, predicting disease that predicts disease that may occur in the future in the subject of disease prediction, based on the non self-examination value information and the self-examination value information, to generate disease prediction information, and outputting information that outputs the disease prediction information, which are executed by a computer.

A program for causing a computer to execute the following procedures according to an example aspect of the invention includes an information acquisition procedure that acquires past non self-examination value information and current self-examination value information of a subject of disease prediction, a disease prediction procedure that predicts disease that may occur in the future in the subject of disease prediction, based on the non self-examination value information and the self-examination value information, to generate disease prediction information, and an information output procedure that outputs the disease prediction information.

A computer-readable recording medium recorded with a program for causing a computer to execute the following procedures according to an example aspect of the invention includes an information acquisition procedure that acquires past non self-examination value information and current self-examination value information of a subject of disease prediction, an disease prediction procedure that predicts disease that may occur in the future in the subject of disease prediction, based on the non self-examination value information and the self-examination value information, to generate disease prediction information, an information output procedure that outputs the disease prediction information.

### Advantageous Effects of Invention

An example advantage according to the present invention is that it becomes possible to find a risk of disease onset even on a daily basis, and to increase an individual awareness of health management.

### BRIEF DESCRIPTION OF DRAWINGS

[FIG. 1] FIG. 1 is a block diagram showing an example configuration of the disease prediction apparatus according to a first example embodiment.
[FIG. 2] FIG. 2 is a block diagram showing an example hardware configuration of the disease prediction apparatus according to the first example embodiment.
[FIG. 3] FIG. 3 is a flowchart showing an example processing in the disease prediction apparatus according to the first example embodiment.
[FIG. 4] FIG. 4 is a block diagram showing an example configuration of the disease prediction apparatus according to a second example embodiment.
[FIG. 5] FIG. 5 is a flowchart showing an example processing in the disease prediction apparatus according to the second example embodiment.
[FIG. 6] FIG. 6 is a reference diagram showing an example estimation of estimated examination value information in the disease prediction apparatus according to the second example embodiment.
[FIG. 7] FIG. 7 is a reference diagram showing an example of disease prediction procedure in the disease prediction apparatus according to the second example embodiment.

### EXAMPLE EMBODIMENTS

The example embodiments of the present invention are described below with reference to the drawings. It is to be noted, however, that the present invention is by no means limited or restricted by the following example embodiments. In the following drawings, identical parts are indicated with identical reference signs. Regarding the descriptions of the example embodiments, reference can be made to one another unless otherwise stated. Furthermore, the configurations of the example embodiments can be combined unless otherwise stated.

### First Example Embodiment

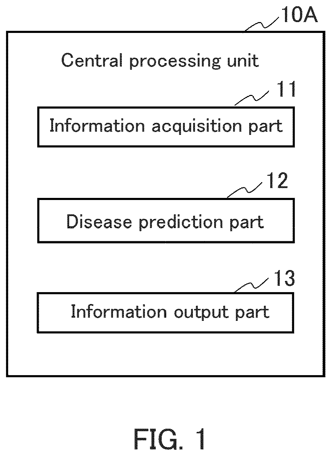

FIG. 1 is a block diagram showing an example configuration of a disease prediction apparatus 10A according to the present example embodiment (hereinafter also referred to as "the apparatus 10A"). As shown in FIG. 1, the apparatus 10A includes an information acquisition part 11, a disease prediction part 12, and an information output part 13. Although not shown, the apparatus 10A may include, for example, an input part, an output part, a display, and/or a storage.

The apparatus 10A may be, for example, one apparatus including the aforementioned parts, or may be an apparatus to which the respective parts are connectable via a communication line network. Further, the apparatus 10A is connectable to an external device described later, via the communication line network. The communication line network is not particularly limited and may be a known network and may be, for example, wired or wireless. Examples of the communication line network include an internet line, WWW (World Wide Web), a telephone line, LAN (Local Area Network), SAN (Storage Area Network), DTN (Delay Tolerant Networking), LPWA (Low Power Wide Area), and L5G (local 5G). Examples of wireless communication include WI-FI^{®} (registered trademark), BLUETOOTH^{®} (registered trademark), local 5G, and LPWA. The wireless communication may be a form where apparatuses directly communicate with each other (Ad-Hoc communication), infrastructure communication, or may be indirect communication via an access point or the like. The apparatus 10A may be built in a server of a system, for example. The apparatus 10A may also be, for example, a personal computer (PC, e.g., a desktop PC and a laptop), a smartphone, a tablet terminal, or the like with the program of the present disclosure installed therein. The apparatus 10A may be in a form of, for example, cloud computing, edge computing, or the like such that at least one of the aforementioned parts is on a server and the rest of the parts are on a terminal.

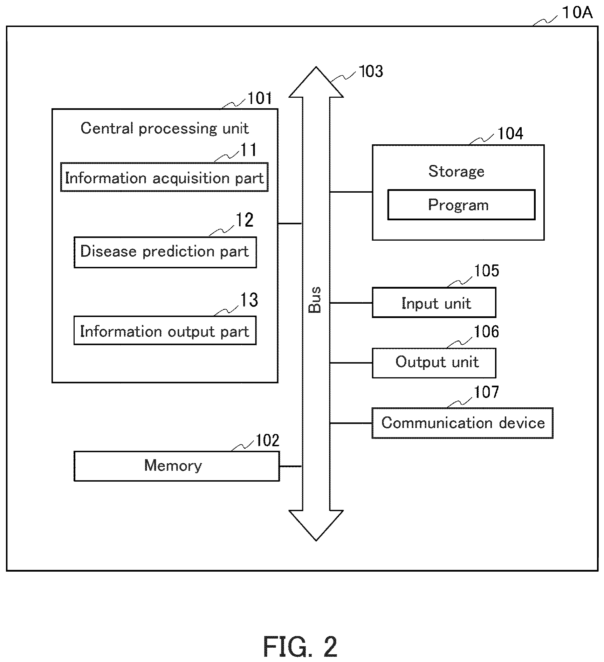

FIG. 2 is a block diagram showing an example hardware configuration of the apparatus 10A. The apparatus 10A includes, for example, a central processing unit (CPU, GPU, etc.) 101, a memory 102, a bus 103, a storage 104, an input unit 105, an output unit 106, a communication device 107, and the like. The units of the apparatus 10A are connected to each other by their respective interfaces (I/F), via the bus 103.

The central processing unit 101 operates in cooperation with other configurations with a controller (a system controller, an I/O controller, or the like), and is responsible for control of the entire apparatus 10A. In the apparatus 10A, the central processing unit 101 executes, for example, the program of the present disclosure or other programs, and reads and writes various data. Specifically, for example, the central processing unit 101 functions as the information acquisition part 11, the disease prediction part 12, and the information output part 13. When the apparatus 10A includes the output part, the central processing unit 101 may function as the output part. The apparatus 10A may include another arithmetic unit such as a CPU, a GPU (Graphics Processing Unit) and an APU (Accelerated Processing Unit), or a combination of these and the CPU.

The bus 103 is connectable to an external device, for example. Examples of the external device include a terminal of a user, an external storage (external data base, etc.), a printer, an external input unit, an external display, and an external imaging device. The apparatus 10A can be, for example, connected to an external network (the communication line network) through the communication device 107 connected to the bus 103, and can be connected to other devices via the external network.

The memory 102 may be, for example, a main memory (main storage). When the central processing unit 101 executes processing, for example, the memory 102 reads various operation programs such as the program of the present disclosure stored in the storage 104 to be described later, and the central processing unit 101 receives the data from the memory 102 to execute the program. The main memory is, for example, a RAM (Random-Access Memory). The memory 102 may be, for example, a ROM (Read-Only Memory).

The storage 104 is also referred to as a so-called auxiliary storage with respect to the main memory (main storage), for example. As mentioned above, the storage 104 stores operation programs including the program of the present disclosure. The storage 104 may be, for example, a combination of a recording medium and a drive for performing reading and writing on a recording medium. The recording medium is not particularly limited and may be a built-in type or an external type, and examples thereof include HD (Hard Disk), CD-ROM, CD-R, CD-RW, MO, DVD, a flash memory, and a memory card. The storage 104 may be, for example, a hard disk drive (HDD) or a solid state drive (SSD), in which a recording medium and a drive are integrated. When the apparatus 10A includes the storage, for example, the storage 104 functions as the storage. The storage can store, for example, past non self-examination value information and current self-examination value information, and the like, which will be described later.

In the apparatus 10A, the memory 102 and the storage 104 can also store various information such as log information, information acquired from an external data base (not shown) or an external device, information generated by the apparatus 10A, and information used when the apparatus 10A executes processing. In this case, the memory 102 and the storage 104 may store, for example, the past non self-examination value information. It should be noted that, at least a piece of the information may be, for example, stored in an external server other than the memory 102 and the storage 104, or may be dispersedly stored in a plurality of terminals using a blockchain technique or the like.

The apparatus 10A further includes, for example, an input unit 105 and an output unit 106. Examples of the input unit 105 include a pointing device such as a touch panel, a track pad, and a mouse; a keyboard; an imaging device such as a camera and a scanner; a card reader such as an IC card reader and a magnetic card reader; and an audio inputting device such as a microphone. Examples of the output unit 106 include a display such as an LED display and a liquid crystal display; an audio output device such as a speaker; and a printer. In the first example embodiment, the input unit 105 and the output unit 106 are configured separately, but the input unit 105 and the output unit 106 may be configured integrally like a touch panel display.

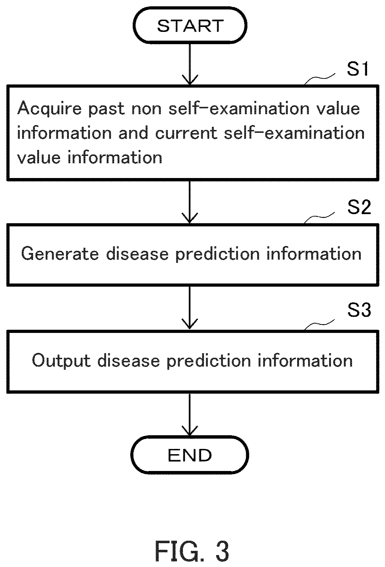

Next, an example of the disease prediction method according to the present example embodiment will be described with reference to the flow chart of FIG.3. The disease prediction method according to the present example embodiment may be performed, for example, as follows using the apparatus 10A of FIG. 1 or FIG. 2. Note that, the disease prediction method according to the present example embodiment is not limited to the use of the apparatus 10A of FIG. 1 or FIG. 2.

First, the information acquisition part 11 acquires past non self-examination value information and current self-examination value information of a subject of disease prediction (S1, acquiring information). The self-examination value information refers to information on examination value measured by the subject of disease prediction for examination items that can be routinely measured by the subject of disease prediction, such as weight, blood pressure, BMI, and self-reported medical inquiry. The self-examination value information may also include attribute information of the subject of disease prediction, such as sex and age. The non self-examination value information is information on examination value other than the self-examination value information, and is, for example, examination value obtained though health checkups or the like including items that cannot be measured by the subject of disease prediction on their own, such as blood test value and urine test value. The non self-examination value information is not limited to examination value obtained though health checkups, and may include, for example, information on results from complete medical checkups, individual diagnostic results, and the like. Further, the non self-examination value information is not limited to actual past examination value, and may be examination value estimated from trends of examination value, passage of time, correlation with self-examination value information, or the like. Methods for acquiring the past non self-examination value information and the current self-examination value information are, for example, acquisition from an external database or direct input to an input device, but are not limited thereto. The methods may be in any form as long as the aforementioned information can be acquired, and may be reception from an external terminal via a communication line, and the like.

Next, the disease prediction part 12 predicts disease that may occur in the future in the subject of disease prediction, based on the non self-examination value information and the self-examination value information, and generates disease prediction information (S2, predicting disease). The likelihood of disease onset is usually predicted from non self-examination value obtained thorough health checkups or the like. However, it is usual to take such checkups only once or twice a year. For this, the self-examination value information measured daily by the subject of disease prediction is used in combination with the non self-examination value information to predict the likelihood of disease onset in daily life outside of health checkup periods. Disease that may occur in the future is predicted based on the non self-examination value information and the self-examination value information, for example, from the correlation between each examination value and disease. For example, disease onset prediction models or the like may be used to predict disease. Note that, the disease prediction information is not limited to the likelihood of disease onset, and may include prediction of the health condition of the subject of disease prediction.

Next, the information output part 13 outputs the disease prediction information (S3, outputting information). The disease prediction information is outputted, for example, by being displayed on a dedicated monitor connected as an external device. The output is not limited thereto, and for example, may be transmission to a terminal of the subject of disease prediction via a communication line, or output to an external database, and may be arbitrarily set depending on the application.

The apparatus 10A according to the present example embodiment can predict disease that may occur in the future based on the non self-examination value information and the self-examination value information. Therefore, the use of the apparatus 10A according to the present example embodiment allows disease prediction, for example, even in daily life regardless of the period of health checkups or the like. This leads to the rise in the individual awareness of health management.

### Second Example Embodiment

The second example embodiment is another example of the disease prediction apparatus of the present disclosure.

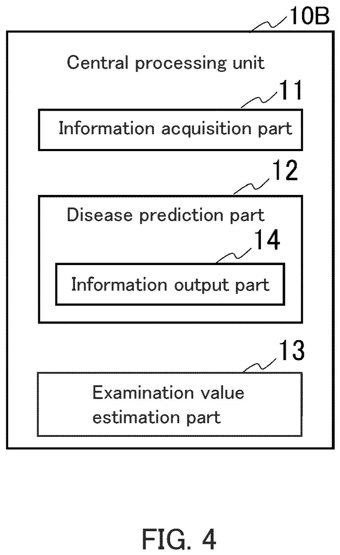

The disease prediction apparatus according to the present example embodiment will be described with reference to FIG. 4. FIG. 4 is a block diagram showing an example configuration of a disease prediction apparatus 10B according to the present example embodiment (hereinafter also referred to as "the apparatus 10B"). As shown in FIG. 4, in the apparatus 10B according to the present example embodiment, among the components of the apparatus 10A according to the first example embodiment (i.e., the information acquisition part 11, the disease prediction part 12, and the information output part 13), the disease prediction part 12 includes, for example, an examination value estimation part 14. The configuration, including the hardware configuration, of the apparatus 10B, is the same as that of the apparatus 10A according to the first example embodiment, except that the disease prediction part 12 includes, for example, the examination value estimation part 14, and the description thereof can be applied here.

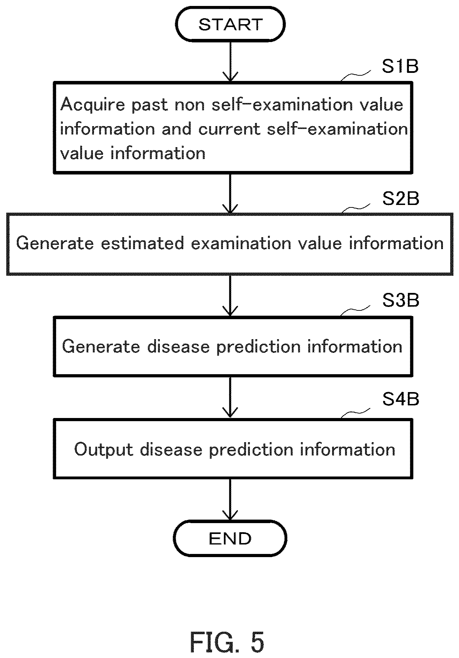

Next, an example of the disease prediction method according to the present example embodiment will be described with reference to the flowchart of FIG. 5. The disease prediction method according to the present example embodiment is performed, for example, as follows using the apparatus 10B shown in FIG. 4. Note that, the disease prediction method according to the present example embodiment is not limited to the use of the apparatus 10B in FIG. 4.

The information acquisition part 11 of the apparatus 10B according to the present example embodiment acquires the past non self-examination value information and the current self-examination value information of the subject of disease prediction (S1B, acquiring information). The process of the acquiring information is the same as in the first example embodiment, and the description thereof can be applied here.

Next, the examination value estimation part 14 in the disease prediction part 12 estimates the current examination value based on at least one of the past non self-examination value information or the self-examination value information, and generates estimated examination value information (S2B, estimating examination value). As described above, it is usual to take health checkups or the like once or twice a year. This causes disease prediction conducted in periods other than the periods of health checkups or the like to use the non self-examination information obtained before the disease prediction. In response to this, estimation of the current examination value for the non self-examination information allows more accurate disease prediction. The examination value estimation part 14 estimates, for example, the current examination value from the trends of the past non self-examination information. The examination value may be estimated, for example, based on the correlation between the past non self-examination information and the self-examination value information. The past non self-examination information used for the analysis of the correlation may be the past examination value as it is, or an estimated value. The estimation of the examination value is not limited to the examples mentioned above, and is performed using at least one of the past non self-examination information or the self-examination value information.

As an example of the estimation of the examination value, the examination value estimation part 14 may generate estimated examination value information by generating a fitted line for predicting variation in the non self-examination value from a plurality of pieces of the past non self-examination value information, and estimating and generating the current examination value based on the fitted line. The fitted line is a line representing predicted values based on a plurality the past examination values, and may be, for example, a fitted curve, a fitted straight line, a fitted polygonal line, or the like.

As another example, the examination value estimation part 14 may generate the estimated examination value information by estimating the current examination value based on the correlation between the non self-examination value information and the self-examination value information, and generating the estimated current examination value.

Next, the disease prediction part 12 predicts disease that may occur in the future in the subject of disease prediction, based on the estimated examination value information generated by the examination value estimation part 14 and the self-examination value information, and generates disease prediction information (S3B, predicting disease). The prediction of disease is the same as in the first example embodiment except that the estimated examination value information generated by the examination value estimation part 14 is used, and the description thereof can be applied here.

Next, the information output part 13 outputs the disease prediction information (S4B, outputting information). The outputting information is the same as in the first example embodiment, and the description thereof can be applied here.

Hereinafter, an example of specific processing of the disease prediction using the apparatus 10B will be described. Note that, the following description is an example of a specific flow, and as described above, the apparatus 10B and the disease prediction method according to the present example embodiment are not limited to the following description. The disease prediction method according to the present example embodiment is not limited to the use of the apparatus 10B.

As described above, the likelihood of disease onset can be predicted based on examination value measured through health checkups or the like. In the prediction, examination value that can be measured by the subject of disease prediction can be replaced with examination value from health checkups or the like, and the examination value from health checkups or the like can be replaced with the current estimated value. Examples of the examination items used for predicting the likelihood of disease onset include basic information (age, sex, etc.), blood tests (LDL, HDL, etc.), medical inquiry information (smoking, diet, etc.), and daily measurable items (blood pressure, weight, BMI, etc.). These items are all inquired, examined, and measured in health checkups or the like. Among these items, only blood tests are difficult for the subject of disease prediction to measure on their own. In response to this, estimation of the current examination value of blood tests using other examination values can realize prediction of the likelihood of disease onset similar to that performed through health checkups or the like.

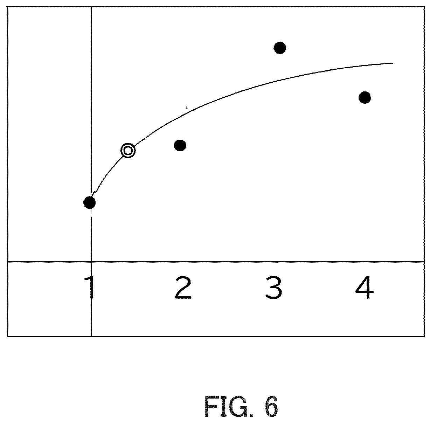

In a case where trends of blood test value variation can be determined based on examination value from a plurality of past blood test values, a fitted curve can be generated to estimate current examination value. FIG. 6 is a diagram illustrating a fitted curve generated from past examination values. The black circles in FIG. 6 represent past actual values. The horizontal axis of the table represents the passage of time, allowing the actual values corresponding to the passage of time to be identified. The curve in FIG. 6 is a fitted curve generated based on past actual values. The use of the fitted curve allows grasp of the rate of variation in examination values, based on the elapsed time from the previous health checkup or the like. The double circle in FIG. 6 represents the corresponding portion of the fitted curve based on the elapsed time from the previous health checkup. This allows estimation of current examination value using the examination value from the previous health checkup. Note that, the estimation of the current examination value based on a plurality of pieces of the past non self-examination value information is not limited to blood tests, and the fitted line used for the analysis is not limited to a fitted curve. In this way, the examination value estimation part 14 generates a fitted line for predicting variation in the non self-examination value from a plurality of pieces of the past non self-examination value information, and estimates and generates the current examination value based on the fitted line.

Further, the correlation between examination value that can be measured by the subject of disease prediction and examination value of blood tests is analyzed. It is considered that the examination value of blood tests may be affected by, for example, age, sex, change in lifestyle, blood pressure, increase or decrease in weight, and the like. The current examination value can be estimated based on, for example, the correlation between statistical tendencies and examination value measured by the subject of disease prediction, such as age. Note that, as the examination value of blood tests, past examination value may be used as it is, or estimated value may be used. The estimation of the current examination value is not limited to the blood tests, as described above.

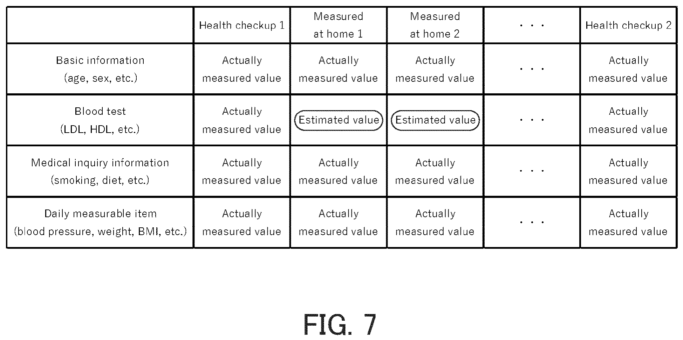

As described above, for a test item with no examination value measured by the subject of disease prediction as the current self-examination value information, the current examination value is estimated based on at least one of the past non self-examination information or the current self-examination value information, to predict the likelihood of disease onset based on the estimated examination value information and the current self-examination value information. FIG. 7 shows an example of the examination values used for the prediction. The table of FIG. 7 shows the passage of time toward the right, and whether each examination item is an actually measured value or an estimated value. The likelihood of disease onset at the time point shown in the header row is predicted based on the examination values shown in the corresponding column.

The apparatus 10B according to the present example embodiment estimates the current information for a test item with no examination value measured by the subject of disease prediction, allowing predictions based on information that captures current situation more accurately.

### Third Example Embodiment

The program according to the present example embodiment is a program for causing a computer to execute each of the processes of the disease prediction method described above. Specifically, the program according to the present example embodiment is a program for causing a computer to execute, for example, an information acquisition procedure, a disease prediction procedure, and an information output procedure.

The information acquisition procedure acquires past non self-examination value information and current self-examination value information of a subject of disease prediction. The disease prediction procedure predicts disease that may occur in the future in the subject of disease prediction, based on the non self-examination value information and the self-examination value information, and generates disease prediction information. The information output procedure outputs the disease prediction information.

The program according to the present example embodiment can also be regarded as a program that causes a computer to function as, for example, the information acquisition procedure, the disease prediction procedure, and the information output procedure.

The program according to the present example embodiment can incorporate the descriptions given in the disease prediction apparatus and the disease prediction method of the present disclosure. In each of the processes, for example, "procedure" and "processing" can be interchangeably used. The program according to the present example embodiment may be recorded in a computer-readable recording medium, for example. The recording medium is, for example, a non-transitory computer-readable recording medium (storage medium). The recording medium is not particularly limited and examples thereof include a random access memory (RAM), a read only memory (ROM), a hard disk (HD), a flash memory (e.g., SSD (Solid State Drive), a USB flash memory, and a SD/SDHC card), an optical disc (e.g., CD-R/CD-RW, DVD-R/DVD-RW, and BD-R/BD-RE), a magneto-optical disk (MO), and a FLOPPY^{®} (registered trademark) disk (FD). The program (may be referred to as a programming product or a program product, for example) according to the present example embodiment may be in a form to be distributed from an external computer, for example. The "distribution" may be distribution via a communication line network or distribution via a wired-connected device. The program according to the present example embodiment may be executed by being installed in a device where the program is distributed, or may be executed without being installed.

While the invention has been particularly shown and described with reference to example embodiments thereof, the invention is not limited to these embodiments. It will be understood by those of ordinary skill in the art that various changes in form and details may be made therein without departing from the spirit and scope of the present invention as defined by the claims.

### SUPPLEMENTARY NOTE

The whole or part of the example embodiments disclosed above can be described as, but not limited to, the following supplementary notes.

### (Supplementary Note 1)

A disease prediction apparatus, including:
an information acquisition part;
a disease prediction part, and
an information output part, wherein
the information acquisition part acquires past non self-examination value information and current self-examination value information of a subject of disease prediction,
the disease prediction part predicts disease that may occur in the future in the subject of disease prediction, based on the non self-examination value information and the self-examination value information, to generate disease prediction information, and
the information output part outputs the disease prediction information.

### (Supplementary Note 2)

The apparatus according to Supplementary Note 1, wherein
the disease prediction part further includes an examination value estimation part, and
the examination value estimation part estimates current examination value, based on at least one of the past non self-examination information or the self-examination value information, to generate estimated examination value information, and
the disease prediction part predicts disease that may occur in the subject of disease prediction, based on the estimated examination value information and the self-examination value information, to generate disease prediction information.

### (Supplementary Note 3)

The apparatus according to Supplementary Note 2, wherein
the examination value estimation part generates a fitted line for predicting variation in non self-examination value from a plurality of pieces of the past non self-examination value information, and estimates current examination value based on the fitted line to generate estimated examination value information.

### (Supplementary Note 4)

The apparatus according to Supplementary Note 2, wherein
the examination value estimation part estimates current examination value, based on a correlation between the non self-examination value information and the self-examination value information, to generate estimated examination value information.

### (Supplementary Note 5)

A disease prediction method, including:
acquiring information,
predicting disease, and
outputting information, wherein
the acquiring information acquires past non self-examination value information and current self-examination value information of a subject of disease prediction,
the predicting disease predicts disease that may occur in the future in the subject of disease prediction, based on the non self-examination value information and the self-examination value information, to generate disease prediction information, and
the outputting disease outputs the disease prediction information,
which are executed by a computer.

### (Supplementary Note 6)

The method according to Supplementary Note 5, wherein
the predicting disease further includes estimating examination value,
the estimating examination value estimates current examination value, based on at least one of the past non self-examination information or the self-examination value information, to generate estimated examination value information, and
the predicting disease predicts disease that may occur in the future in the subject of disease prediction, based on the estimated examination value information and the self-examination value information, to generate disease prediction information,
which are executed by a computer.

### (Supplementary Note 7)

The method according to Supplementary Note 6, wherein
the estimating examination value generates a fitted line for predicting variation in non self-examination value from a plurality of pieces of the past non-self examination value information, and estimates current examination value based on the fitted line, to generate estimated examination value information,
which is executed by a computer.

### (Supplementary Note 8)

The method according to Supplementary Note 6, wherein
the estimating examination value estimates current examination value, based on a correlation between the non self-examination value information and the self-examination value information, to generate estimated examination value information,
which is executed by a computer.

### (Supplementary Note 9)

A program for causing a computer to execute the following procedures, including:
an information acquisition procedure,
a disease prediction procedure, and
an information output procedure, wherein
the information acquisition procedure acquires past non self-examination value information and current self-examination value information of a subject of disease prediction,
the disease prediction procedure predicts disease that may occur in the future in the subject of disease prediction, based on the non self-examination value information and the self-examination value information, to generate disease prediction information, and
the information output procedure outputs the disease prediction information.

### (Supplementary Note 10)

The program according to Supplementary Note 9 for causing a computer to execute the following procedures, wherein
the disease prediction procedure further includes an examination value estimation procedure, and the examination value estimation procedure estimates current examination value, based on at least one of the past non self-examination information or the self-examination value information, to generate estimated examination value information, and
the disease prediction procedure predicts disease that may occur in the subject of disease prediction, based on the estimated examination value information and the self-examination value information, to generate disease prediction information.

### (Supplementary Note 11)

The program according to Supplementary Note 10 for causing a computer to execute the following procedure, wherein
the examination value estimation procedure generates a fitted line for predicting variation in non self-examination value from a plurality of pieces of the past non self-examination value information, and estimates current examination value based on the fitted line to generate estimated examination value information.

### (Supplementary Note 12)

A program according to Supplementary Note 10 for causing a computer to execute the following procedure, wherein
the examination value estimation procedure estimates current examination value, based on a correlation between the non self-examination value information and the self-examination value information, to generate estimated examination value information.

### (Supplementary Note 13)

A computer-readable recording medium recorded with a program for causing a computer to execute the following procedures, including:
an information acquisition procedure,
an disease prediction procedure, and
an information output procedure, wherein
the information acquisition procedure acquires past non self-examination value information and current self-examination value information of a subject of disease prediction,
the disease prediction procedure predicts disease that may occur in the future in the subject of disease prediction, based on the non self-examination value information and the self-examination value information, to generate disease prediction information, and
the information output procedure outputs the disease prediction information.

### (Supplementary Note 14)

The recording medium according to Supplementary Note 13 recorded with a program for causing a computer to execute the following procedures, wherein
the disease prediction procedure further includes the examination value estimation procedure, and
the examination value estimation procedure estimates current examination value, based on at least one of the past non self-examination information or the self-examination value information, to generate estimated examination value information, and
the disease prediction procedure predicts disease that may occur in the subject of disease prediction, based on the estimated examination value information and the self-examination value information, to generate disease prediction information.

### (Supplementary Note 15)

The recording medium according to Supplementary Note 14 recorded with a program for causing a computer to execute the following procedure, wherein
the examination value estimation procedure generates a fitted line for predicting variation in non self-examination value from a plurality of pieces of the past non self-examination value information, and estimates current examination value based on the fitted line to generate estimated examination value information.

### (Supplementary Note 16)

The recording medium according to Supplementary Note 14 recorded with a program for causing a computer to execute the following procedure, wherein
the examination value estimation procedure estimates current examination value, based on a correlation between the non self-examination value information and the self-examination value information, to generate estimated examination value information.

### Industrial Applicability

An example advantage according to the present invention is that it becomes possible to find a risk of disease onset even on a daily basis, and to increase an individual awareness of health management.

### Reference Signs List

- 10A, 10B:: disease prediction apparatus
- 11:: information acquisition part
- 12:: disease prediction part
- 13:: information output part
- 14:: examination value estimation part
- 101:: central processing unit
- 102:: memory
- 103:: bus
- 104:: storage
- 105:: input unit
- 106:: output unit
- 107:: communication device

## Claims

1. A disease prediction apparatus, comprising:
an information acquisition part;
a disease prediction part, and
an information output part, wherein
the information acquisition part acquires past non self-examination value information and current self-examination value information of a subject of disease prediction,
the disease prediction part predicts disease that may occur in the future in the subject of disease prediction, based on the non self-examination value information and the self-examination value information, to generate disease prediction information, and
the information output part outputs the disease prediction information.

2. The apparatus according to claim 1, wherein
the disease prediction part further includes an examination value estimation part, and
the examination value estimation part estimates current examination value, based on at least one of the past non self-examination information or the self-examination value information, to generate estimated examination value information, and
the disease prediction part predicts disease that may occur in the subject of disease prediction, based on the estimated examination value information and the self-examination value information, to generate disease prediction information.

3. The apparatus according to claim 2, wherein
the examination value estimation part generates a fitted line for predicting variation in non self-examination value from a plurality of pieces of the past non self-examination value information, and estimates current examination value based on the fitted line to generate estimated examination value information.

4. The apparatus according to claim 2, wherein
the examination value estimation part estimates current examination value, based on a correlation between the non self-examination value information and the self-examination value information, to generate estimated examination value information.

5. A disease prediction method, comprising:
acquiring information,
predicting disease, and
outputting information, wherein
the acquiring information acquires past non self-examination value information and current self-examination value information of a subject of disease prediction,
the predicting disease predicts disease that may occur in the future in the subject of disease prediction, based on the non self-examination value information and the self-examination value information, to generate disease prediction information, and
the outputting disease outputs the disease prediction information,
which are executed by a computer.

6. The method according to claim 5, wherein
the predicting disease further includes estimating examination value,
the estimating examination value estimates current examination value, based on at least one of the past non self-examination information or the self-examination value information, to generate estimated examination value information, and
the predicting disease predicts disease that may occur in the future in the subject of disease prediction, based on the estimated examination value information and the self-examination value information, to generate disease prediction information,
which are executed by a computer.

7. The method according to claim 6, wherein
the estimating examination value generates a fitted line for predicting variation in non self-examination value from a plurality of pieces of the past non-self examination value information, and estimates current examination value based on the fitted line, to generate estimated examination value information,
which is executed by a computer.

8. The method according to claim 6, wherein
the estimating examination value estimates current examination value, based on a correlation between the non self-examination value information and the self-examination value information, to generate estimated examination value information,
which is executed by a computer.

9. A program for causing a computer to execute the following procedures, comprising:
an information acquisition procedure,
a disease prediction procedure, and
an information output procedure, wherein
the information acquisition procedure acquires past non self-examination value information and current self-examination value information of a subject of disease prediction,
the disease prediction procedure predicts disease that may occur in the future in the subject of disease prediction, based on the non self-examination value information and the self-examination value information, to generate disease prediction information, and
the information output procedure outputs the disease prediction information.

10. The program according to claim 9 for causing a computer to execute the following procedures, wherein
the disease prediction procedure further includes an examination value estimation procedure, and
the examination value estimation procedure estimates current examination value, based on at least one of the past non self-examination value information or the self-examination value information, to generate estimated examination value information, and
the disease prediction procedure predicts disease that may occur in the subject of disease prediction, based on the estimated examination value information and the self-examination value information, to generate disease prediction information.

11. The program according to claim 10 for causing a computer to execute the following procedure, wherein
the examination value estimation procedure generates a fitted line for predicting variation in non self-examination value from a plurality of pieces of the past non self-examination information, and estimates current examination value based on the fitted line to generate estimated examination value information.

12. A program according to claim 10 for causing a computer to execute the following procedure, wherein
the examination value estimation procedure estimates current examination value, based on a correlation between the non self-examination value information and the self-examination value information, to generate estimated examination value information.

13. A computer-readable recording medium recorded with a program for causing a computer to execute the following procedures, comprising:
an information acquisition procedure,
a disease prediction procedure, and
an information output procedure, wherein
the information acquisition procedure acquires past non self-examination value information and current self-examination value information of a subject of disease prediction,
the disease prediction procedure predicts disease that may occur in the future in the subject of disease prediction, based on the non self-examination value information and the self-examination value information, to generate disease prediction information, and
the information output procedure outputs the disease prediction information.

14. The recording medium according to claim 13 recorded with a program for causing a computer to execute the following procedures, wherein
the disease prediction procedure further includes the examination value estimation procedure, and
the examination value estimation procedure estimates current examination value, based on at least one of the past non self-examination information or the self-examination value information, to generate estimated examination value information, and
the disease prediction procedure predicts disease that may occur in the subject of disease prediction, based on the estimated examination value information and the self-examination value information, to generate disease prediction information.

15. The recording medium according to claim 14 recorded with a program for causing a computer to execute the following procedure, wherein
the examination value estimation procedure generates a fitted line for predicting variation in non self-examination value from a plurality of pieces of the past non self-examination value information, and estimates current examination value based on the fitted line to generate estimated examination value information.

16. The recording medium according to claim 14 recorded with a program for causing a computer to execute the following procedure, wherein
the examination value estimation procedure estimates current examination value, based on a correlation between the non self-examination value information and the self-examination value information, to generate estimated examination value information.
